(19) Europäisches Patentamt
European Patent Office
Office européen des brevets

(11) Veröffentlichungsnummer: **0 499 043 A1**

# (12) EUROPÄISCHE PATENTANMELDUNG

(21) Anmeldenummer: **92100715.9**

(51) Int. Cl.5: **C07D 213/20**

(22) Anmeldetag: **17.01.92**

(30) Priorität: **09.02.91 DE 4103968**

(43) Veröffentlichungstag der Anmeldung: **19.08.92 Patentblatt 92/34**

(84) Benannte Vertragsstaaten: **DE FR GB IT**

(71) Anmelder: **BASF Aktiengesellschaft**
**Carl-Bosch-Strasse 38**
**W-6700 Ludwigshafen(DE)**

(72) Erfinder: **Houben, Jochen, Dr.**
**Hahnenweide 21**
**W-4154 Toenisvorst 2(DE)**
Erfinder: **Mueller, Josef, Dr.**
**Rieslingstrasse 15**
**W-6711 Grosskarlbach(DE)**
Erfinder: **Oftring, Alfred, Dr.**
**Im Roehrich 49**
**W-6702 Bad Duerkheim(DE)**
Erfinder: **Widder, Rudi, Dr.**
**In der Taesch 7**
**W-6906 Leimen(DE)**

## (54) Verfahren zur Herstellung von Sulfatobetainen.

(57) Herstellung von Sulfatobetainen I

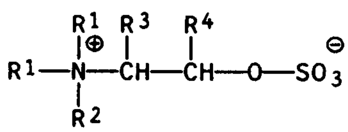

I

bei denen die einzelnen Reste $R^1$ und $R^2$ gleich oder verschieden sind und

- einen gesättigten geradkettigen oder verzweigten Alkylrest mit 1 bis 22 C-Atomen, einen Cycloalkylrest mit 5 bis 7 C-Atomen im Ring, einen Phenyl- oder Naphthylrest oder einen Aralkylrest mit insgesamt 7 bis 12 C-Atomen und oder
- die beiden Reste $R^1$ zusammen mit dem N-Atom einen 5- oder 6-gliedrigen heterocyclischen Ring, der ein oder mehrere weitere Heteroatome und einen anellierten Benzolring enthalten kann, wobei für $R^2$ ein Alkylrest mit 1 bis 4 C-Atomen steht, oder
- die beiden Reste $R^1$ zusammen mit dem N-Atom und dem Rest $R^2$ einen 5- oder 6-gliedrigen ungesättigten heterocyclischen Ring, der ein oder mehrere weitere Heteroatome und einen anellierten Benzolring enthalten kann, bezeichnen und

$R^3$ und $R^4$ gleich oder verschieden sind und $R^3$ ein Wasserstoffatom oder einen Alkylrest mit 1 bis 7 C-Atomen und $R^4$ ein Wasserstoffatom, einen Alkylrest mit 1 bis 20 C-Atomen oder einen Phenylrest bedeuten, wobei die Reste $R^3$ und $R^4$ miteinander vertauscht sein können,
indem man Stickstoffbasen mit einem tertiären N-Atom II

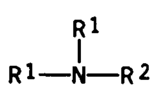

II

EP 0 499 043 A1

mit einem Alkylencarbonat III

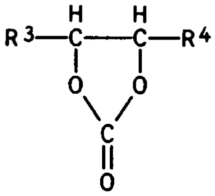

III

und mit Schwefelsäure bei Temperaturen von 80 bis 220 ° C umsetzt.

Die vorliegende Erfindung betrifft ein verbessertes Verfahren zur Herstellung von Sulfatobetainen.

Die DE-A 36 35 230 betrifft ein Verfahren zur Herstellung von Sulfatobetainen, bei dem ein Addukt von Schwefeltrioxid an eine Stickstoffbase II mit einem Alkylencarbonat III bei 80 bis 220 °C umgesetzt wird. Nachteilig hierbei ist jedoch, daß diese Umsetzung erst dann optimal abläuft, wenn entweder überschüssiges Alkylencarbonat III oder die dem $SO_3$-Addukt zugrundeliegende Base II in überschüssiger Menge vorliegt.

Weiterhin ist die Herstellung der gemäß DE-A 36 35 230 eingesetzten $SO_3$-Addukte problematisch, weil das üblicherweise hierzu verwendete Schwefeltrioxid nur sehr schwer handhabbar ist, da es schon mit Wasserspuren explosionsartig reagiert (s. Römpps Chemie-Lexikon, 8. Auflage, S. 3765). Auch mit Stickstoffbasen reagiert Schwefeltrioxid so stark exotherm, daß im technischen Maßstab eine Reaktionskontrolle äußerst schwierig durchzuführen ist; die Reaktionswärme ist nur schwer abführbar, da es bei zu starker Außenkühlung häufig zu einer wärmeisolierenden Belagbildung auf der Innenwand des Reaktionskessels kommt.

Daher war es Aufgabe der vorliegenden Erfindung, ein möglichst einfaches, wirtschaftliches und ungefährliches Herstellungsverfahren für Sulfatobetaine bereitzustellen, welches sich auch gut in einen großtechnischen Maßstab übertragen läßt.

Demgemäß wurde eine Verfahren zur Herstellung von Sulfatobetainen der allgemeinen Formel I

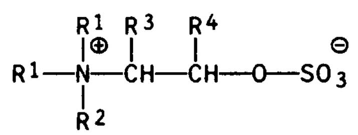

I

in der die einzelnen Reste $R^1$ und $R^2$ gleich oder verschieden sind und

- einen gesättigten geradkettigen oder verzweigten Alkylrest mit 1 bis 22 C-Atomen, einen Cycloalkylrest mit 5 bis 7 C-Atomen im Ring, einen Phenyl- oder Naphthylrest oder einen Aralkylrest mit insgesamt 7 bis 12 C-Atomen oder
- die beiden Reste $R^1$ zusammen mit dem N-Atom einen 5- oder 6-gliedrigen heterocyclischen Ring, der ein oder mehrere weitere Heteroatome und einen anellierten Benzolring enthalten kann, wobei für $R^2$ ein Alkylrest mit 1 bis 4 C-Atomen steht, oder
- die beiden Reste $R^1$ zusammen mit dem N-Atom und dem Rest $R^2$ einen 5- oder 6-gliedrigen ungesättigten heterocyclischen Ring, der ein oder mehrere weitere Heteroatome und einen anellierten Benzolring enthalten kann, bezeichnen und

$R^3$ und $R^4$ gleich oder verschieden sind und $R^3$ ein Wasserstoffatom oder einen Alkylrest mit 1 bis 7 C-Atomen und $R^4$ ein Wasserstoffatom, einen Alkylrest mit 1 bis 20 C-Atomen oder einen Phenylrest bedeuten, wobei die Reste $R^3$ und $R^4$ miteinander vertauscht sein können,
gefunden, welches dadurch gekennzeichnet ist, daß man Stickstoffbasen mit einem tertiären N-Atom der allgemeinen Formel II

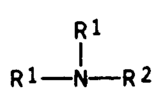

II

mit einem Alkylencarbonat der allgemeinen Formel III

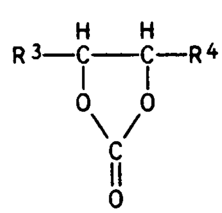

III

und mit Schwefelsäure bei Temperaturen von 80 bis 220 °C umsetzt.

Die am N-Atom der Sulfatobetaine I befindlichen Reste $R^1$ und $R^2$ sind durch die Auswahl der zu deren Herstellung eingesetzten Stickstoffbasen II festgelegt. Als Stickstoffbasen II kommen in Betracht:

- tertiäre aliphatische, cycloaliphatische und aromatische Amine, z.B. Trisdodecylamin, Tristearylamin, Triscyclohexylamin, Triphenylamin, N,N-Dimethyl-N-dodecylamin, N,N-Diethyl-N-phenylamin, N,N-Dimethyl-N-stearylamin, Trimethylamin, Triethylamin, Tributylamin, Tribenzylamin oder N-Benzyl-N,N-dimethylamin; hiervon werden aliphatische Amine mit Alkylresten mit 1 bis 4 C-Atomen für $R^1$ und einem Alkylrest mit 12 bis 18 C-Atomen für $R^2$, beispielsweise N,N-Dimethyl-N-stearylamin oder N,N-Dimethyl-N-dodecylamin, bevorzugt;
- fünf- oder sechsgliedrige heterocyclische Amine, welche am besagten N-Atom eine Alkylgruppe mit 1 bis 4 C-Atomen tragen, z.B. N-Methylpiperidin, N-Ethylpiperidin, N-Methylmorpholin, N-Ethylmorpholin, N-Methylpyrazol, N-Methylimidazol oder N-Methylbenzimidazol; hiervon werden N-Alkylimidazole mit 1 bis 12 C-Atomen im Alkyl, welche an einem der C-Atome zusätzlich durch einen Alkylrest mit 1 bis 6 C-Atomen oder durch Phenyl substituiert sein können, beispielsweise N-Methylimidazol, N-Ethylimidazol, 1,2-Dimethylimidazol, N-Methyl-2-phenylimidazol oder N-Dodecylimidazol, bevorzugt;
- fünf- oder sechsgliedrige, am besagten N-Atom ungesättigte heterocyclische Amine, z.B. Pyridin, Chinolin, Isochinolin, Oxazol, Isoxazol, Acridin, Phenacridin, Pyrazin oder Pyridazin; hiervon werden Pyridin, das durch ein oder zwei Alkylreste mit 1 bis 4 C-Atomen, eine Alkoxycarbonylgruppe mit 1 bis 4 C-Atomen im Alkyl oder eine Nitrilgruppe substituiert sein kann, z.B. $\alpha$-, $\beta$- oder $\gamma$-Picolin, 2-Ethylpyridin, Nicotinsäuremethylester oder Nicotinsäurenitril, sowie Chinolin und Isochinolin, die durch eine Methylgruppe substituiert sein können, z.B. Chinaldin, bevorzugt.

Die 1,2-Alkylencarbonate III sind im Prinzip bekannt und können beispielsweise aus dem entsprechenden Epoxid und Kohlendioxid unter Druck hergestellt werden. Bevorzugt werden als Ausgangsverbindungen III Ethylencarbonat oder 1,2-Propylencarbonat eingesetzt. Es wird darauf hingewiesen, daß Ausgangsverbindungen mit mehreren derartigen cyclischen Carbonatgruppen Di- oder Polysulfatobetaine ergeben.

Die Umsetzung der Verbindungen II mit den Verbindungen III und mit Schwefelsäure erfolgt bei Temperaturen von 80 bis 220 °C, vorzugsweise 100 bis 180 °C, und üblicherweise bei Normaldruck.

In einer bevorzugten Ausführungsform verwendet man für die Umsetzung ein inertes organisches Lösungsmittel, das mit Wasser ein azeotropes Gemisch bildet, um so das bei der Umsetzung entstehende Wasser auf einfache Weise zu entfernen ("auszuschleppen"). In Frage kommen hierfür beispielsweise aromatische Kohlenwasserstoffe wie Toluol und Xylol, aliphatische Kohlenwasserstoffe wie n-Heptan oder Methylcyclohexan, Ketone wie Methyl-isobutylketon oder Cyclohexanon oder chlorierte aromatische Kohlenwasserstoffe wie Chlorbenzol.

Das Molverhältnis von Stickstoffbase II zu Alkylencarbonat III bei der erfindungsgemäßen Umsetzung beträgt in der Regel 1 : 0,8 bis 1 : 2, vorzugsweise 1 : 0,9 bis 1 : 1,2, besonders bevorzugt 1 : 0,95 bis 1 : 1,05, insbesondere 1 : 1. Dabei beträgt die benötigte Menge an Schwefelsäure zweckmäßigerweise 1 bis 2 mol pro Mol III, vorzugsweise 1 bis 1,3 mol pro Mol III. Die Schwefelsäure wird üblicherweise als konzentrierte Säure eingesetzt, sie hat dabei einen Restwassergehalt von 0 bis 5 Gew.-%.

Die Gegenwart von freier Stickstoffbase II im Reaktionsgemisch führt zu einer Beschleunigung der Umsetzung. Wegen dieses Effektes empfiehlt es sich, die erfindungsgemäße Umsetzung so zu führen, daß man die gesamte Menge der Stickstoffbase II mit der gesamten Menge des Alkylencarbonats III und 0,1 bis 0,5 mol Schwefelsäure pro Mol III vorlegt und die restliche Menge der Schwefelsäure im Laufe der Umsetzung kontinuierlich zugibt, bis das oben genannte Molverhältnis von Schwefelsäure zu Alkylencarbonat III erreicht ist (Ausführungsform A).

Zweckmäßigerweise wird hierbei zunächst die Base II in Abwesenheit eines organischen Lösungsmittels mit dem Alkylencarbonat III und einem Teil der Schwefelsäure umgesetzt, anschließend wird durch Zugabe des Restes der Schwefelsäure und des organischen Lösungsmittels unter Wasserabscheidung die Umsetzung zum Endprodukt I vorgenommen. Ergibt die Mischung der Base II mit dem Alkylencarbonat III und der Schwefelsäure keine flüssige Phase, ist es vorteilhafter, daß man das Lösungsmittel von Anfang an zusetzt. Die vollständige Umsetzung zum Endprodukt I ist erreicht, wenn sich kein Wasser mehr abscheidet.

In einer weiteren bevorzugten Ausführungsform legt man die gesamte Menge des Alkylencarbonats III mit 0,15 bis 0,95 mol, vorzugsweise 0,3 bis 0,7 mol Stickstoffbase II und 0,1 bis 0,5 mol Schwefelsäure pro Mol III vor und gibt die restlichen Mengen der Stickstoffbase II und der Schwefelsäure im Laufe der Umsetzung kontinuierlich oder portionsweise zu, bis die oben genannten Molverhältnisse von Stickstoffbase II zu Alkylencarbonat III und von Schwefelsäure zu Alkylencarbonat III erreicht sind (Ausführungsform B). Bei dieser Vorgehensweise ist es zweckmäßig, das Lösungsmittel von Anfang an zuzusetzen und das gebildete Wasser sofort durch azeotrope Destillation zu entfernen.

Die häufig kristallin anfallenden Sulfatobetaine I können in einfacher Weise durch Absaugen isoliert werden. Nach dem Waschen mit einem Lösungsmittel wie Ethanol oder Methanol werden praktisch analysenreine Produkte in hohen Ausbeuten erhalten.

Man kann aber auch nach Vervollständigung der Umsetzung zum Reaktionsgemisch Wasser geben,

wobei man durch Zusatz einer Hilfsbase den pH-Wert oberhalb 2, besser oberhalb 3, hält. Nach dem Abtrennen des organischen Lösungsmittels kann durch Zusatz eines polaren wassermischbaren Lösungsmittels wie Methanol oder Ethanol das Produkt kristallin ausgefällt werden.

Die nach dem erfindungsgemäßen Verfahren hergestellten Verbindungen I können als Tenside, insbesondere als Wasch- und Hilfsmittel auf dem Textilgebiet, als Galvanisierungshilfsmittel und als Reinigungsmittel verwendet werden. Sie sind z.B. Ausgangsstoffe für Waschmittel, Netzmittel, Emulgatoren und Dispergatoren.

Durch das erfindungsgemäße Verfahren werden insbesondere heterocyclische Ethanosulfate, die als Galvanisierhilfsmittel hervorragende Spitzenglanzbildner sind, leicht zugänglich. Hiervon sind beispielsweise zu nennen das 2-Pyridinium-1-sulfatoethan oder das 2-Chinolinium-1-sulfatoethan.

Die erfindungsgemäße Umsetzung kann für den Fall der Verwendung von Pyridin und Umsetzung mit Ethylencarbonat und Schwefelsäure durch folgende Gleichung wiedergegeben werden:

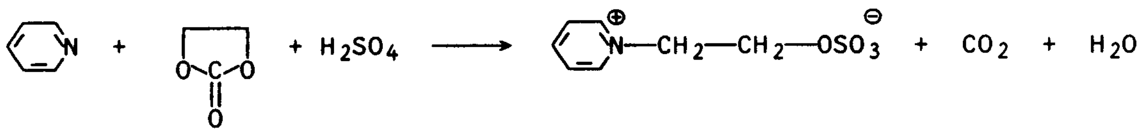

Im Vergleich zum in der DE-A 36 35 230 beschriebenen Verfahren zeichnet sich das erfindungsgemäße Verfahren dadurch aus, daß es einfacher und wirtschaftlicher ist, da kein überschüssiges teures Alkylencarbonat als Lösungsmittel benötigt wird und auch die Stickstoffbase nicht im Überschuß eingesetzt zu werden braucht, d.h. die Ausbeute, bezogen auf eingesetzte Base II, ist beim erfindungsgemäßen Verfahren demgemäß höher. Auch ist die Umsetzung hier einfacher zu kontrollieren, da die Gefahr des Ablaufes von Neben- oder Folgereaktionen, wie beispielsweise beim Verfahren der DE-A 36 35 230 eine Verfärbung des Endproduktes, gering ist.

Ein weiterer wesentlicher Vorteil des erfindungsgemäßen Verfahrens ist der Verzicht auf Schwefeltrioxid als Ausgangsmaterial für die Synthese. Die stattdessen eingesetzte Schwefelsäure ist ungefährlicher und somit einfacher zu handhaben. Zudem ist bei Schwefelsäure als Flüssigkeit im Gegensatz zu Schwefeltrioxid als Feststoff das Problem der schlechten Dosierbarkeit nicht mehr vorhanden. Vor allem aber entfällt das Problem der schwierigen Reaktionskontrolle bei der stark exothermen Umsetzung von Stickstoffbasen mit Schwefeltrioxid.

Beispiel 1

Herstellung von 2-Pyridinium-1-sulfatoethan gemäß Ausführungsform A

Eine Mischung aus 158 g (2,0 mol) Pyridin, 176 g (2,0 mol) Ethylencarbonat und 51 g (0,5 mol) konz. Schwefelsäure wurde unter Rühren auf 125 °C aufgeheizt und bei dieser Temperatur wurden unter Rückfluß innerhalb von 4 Stunden kontinuierlich weitere 128 g (1,3 mol) konz. Schwefelsäure zugetropft. Danach wurde noch 2 Stunden bei 125 °C weitergerührt. Anschließend wurden weitere 30 g (0,3 mol) konz. Schwefelsäure sowie 260 g Toluol zugegeben und das Wasser wurde durch azeotrope Destillation mit Toluol am Wasserabscheider entfernt. Nach 16 Stunden hatten sich 42 g Wasser abgeschieden.

Nach Abkühlung wurde das Produkt abfiltriert und mit 200 g Methanol gewaschen. Man erhielt 377 g der Titelverbindung, entsprechend einer Ausbeute von 93 %, in Form eines farblosen Kristallpulvers vom Schmelzpunkt 203-205 °C. Der titrimetrisch ermittelte Gehalt an Pyridiniumsulfat als Nebenprodukt lag unter 0,05 Gew.-%.

Beispiel 2

Herstellung von 2-Pyridinium-1-sulfatoethan gemäß Ausführungsform B

Eine Mischung aus 79 g (1,0 mol) Pyridin, 176 g (2,0 mol) Ethylencarbonat, 30 g (0,3 mol) konz. Schwefelsäure und 60 g Toluol wurde unter Rühren auf 120 °C aufgeheizt und es wurde begonnen, gebildetes Wasser durch azeotrope Destillation am Wasserabscheider zu entfernen. Gleichzeitig wurden bei dieser Temperatur innerhalb von 7 Stunden weitere 82 g (1,04 mol) Pyridin und weitere 61 g (0,6 mol) konz. Schwefelsäure zugetropft. Anschließend wurde noch eine Stunde unter Rückfluß erhitzt. Das bis dahin entfernte Wasser (25 g) enthielt ca. 16 Gew.-% Pyridin. Im Anschluß wurden weitere 13 g (0,13 mol) konz.

Schwefelsäure und weitere 100 g Toluol zugegeben und durch 10-stündige azeotrope Destillation wurden weitere 18 g Wasser (pyridinfrei) entfernt.

Nach Abkühlung wurde das Produkt abfiltriert und mit 150 g Methanol gewaschen. Man erhielt 389 g der Titelverbindung, entsprechend einer Ausbeute von 96 %, in Form eines farblosen Kristallpulvers vom Schmelzpunkt 203-205 ° C. Der titrimetrisch ermittelte Gehalt an Pyridiniumsulfat lag unter 0,05 Gew.-%.

**Patentansprüche**

**1.** Verfahren zur Herstellung von Sulfatobetainen der allgemeinen Formel I

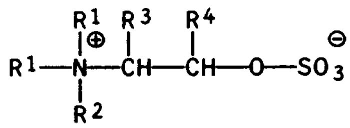

I

in der die einzelnen Reste $R^1$ und $R^2$ gleich oder verschieden sind und
- einen gesättigten geradkettigen oder verzweigten Alkylrest mit 1 bis 22 C-Atomen, einen Cycloalkylrest mit 5 bis 7 C-Atomen im Ring, einen Phenyl- oder Naphthylrest oder einen Aralkylrest mit insgesamt 7 bis 12 C-Atomen oder
- die beiden Reste $R^1$ zusammen mit dem N-Atom einen 5- oder 6-gliedrigen heterocyclischen Ring, der ein oder mehrere weitere Heteroatome und einen anellierten Benzolring enthalten kann, wobei für $R^2$ ein Alkylrest mit 1 bis 4 C-Atomen steht, oder
- die beiden Reste $R^1$ zusammen mit dem N-Atom und dem Rest $R^2$ einen 5- oder 6-gliedrigen ungesättigten heterocyclischen Ring, der ein oder mehrere weitere Heteroatome und einen anellierten Benzolring enthalten kann, bezeichnen und

$R^3$ und $R^4$ gleich oder verschieden sind und $R^3$ ein Wasserstoffatom oder einen Alkylrest mit 1 bis 7 C-Atomen und $R^4$ ein Wasserstoffatom, einen Alkylrest mit 1 bis 20 C-Atomen oder einen Phenylrest bedeuten, wobei die Reste $R^3$ und $R^4$ miteinander vertauscht sein können,

dadurch gekennzeichnet, daß man Stickstoffbasen mit einem tertiären N-Atom der allgemeinen Formel II

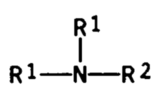

II

mit einem Alkylencarbonat der allgemeinen Formel III

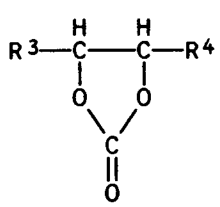

III

und mit Schwefelsäure bei Temperaturen von 80 bis 220 ° C umsetzt.

**2.** Verfahren zur Herstellung von Sulfatobetainen I nach Anspruch 1, dadurch gekennzeichnet, daß man als Stickstoffbase II
- ein aliphatisches Amin mit Alkylresten mit 1 bis 4 C-Atomen für $R^1$ und einem Alkylrest mit 12 bis 18 C-Atomen für $R^2$,
- ein N-Alkyl-imidazol mit 1 bis 12 C-Atomen im Alkyl, das an einem der C-Atome zusätzlich durch einen Alkylrest mit 1 bis 6 C-Atomen oder durch Phenyl substituiert sein kann,
- oder Pyridin, das durch ein oder zwei Alkylreste mit 1 bis 4 C-Atomen, eine Alkoxycarbonylgruppe mit 1 bis 4 C-Atomen im Alkyl oder eine Nitrilgruppe substituiert sein kann, oder Chinolin oder

Isochinolin, die durch eine Methylgruppe substituiert sein können,
einsetzt.

3. Verfahren zur Herstellung von Sulfatobetainen nach Anspruch 1 oder 2, dadurch gekennzeichnet, daß man als Alkylencarbonat III Ethylencarbonat oder 1,2-Propylencarbonat einsetzt.

4. Verfahren zur Herstellung von Sulfatobetainen I nach den Ansprüchen 1 bis 3, dadurch gekennzeichnet, daß man hierbei ein inertes Lösungsmittel verwendet, das mit Wasser ein azeotropes Gemisch bildet.

5. Verfahren zur Herstellung von Sulfatobetainen I nach den Ansprüchen 1 bis 4, dadurch gekennzeichnet, daß man die gesamte Menge der Stickstoffbase II mit der gesamten Menge des Alkylencarbonats III und 0,1 bis 0,5 mol Schwefelsäure pro Mol III vorlegt und die restliche Menge der Schwefelsäure im Laufe der Umsetzung kontinuierlich oder portionsweise zugibt.

6. Verfahren zur Herstellung von Sulfatobetainen I nach den Ansprüchen 1 bis 4, dadurch gekennzeichnet, daß man die gesamte Menge des Alkylencarbonats III mit 0,15 bis 0,95 mol Stickstoffbase II und 0,1 bis 0,5 mol Schwefelsäure pro Mol III vorlegt und die restlichen Mengen der Stickstoffbase II und der Schwefelsäure im Laufe der Umsetzung kontinuierlich oder portionsweise zugibt.

Europäisches Patentamt

# EUROPÄISCHER RECHERCHENBERICHT

Nummer der Anmeldung

EP 92 10 0715

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 264 089 (BASF AKTIENGESELLSCHAFT)<br>* Ansprüche 1-5; Beispiele 1,2 * | 1-6 | C07D213/20 |
| D | & DE-A-3 635 230<br>--- | | |
| A | EP-A-0 282 908 (BASF AKTIENGESELLSCHAFT)<br>* das ganze Dokument *<br>--- | 1-6 | |
| A | DE-A-2 238 371 (UNILEVER N.V.)<br>* das ganze Dokument *<br>----- | 1 | |
| | | | **RECHERCHIERTE SACHGEBIETE (Int. Cl.5)** |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 06 MAI 1992 | P. BOSMA |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)